# FASCICULE DE BREVET EUROPEEN

(11) **EP 1 150 692 B1**
(45) Date de publication et mention de la délivrance du brevet: **17.12.2003**
(21) Numéro de dépôt: 00905120.2
(22) Date de dépôt: 11.02.2000
(51) Int. Cl.: A61K 31/715, A61K 31/728, A61P 35/02

(54) **UTILISATION DE L'ACIDE HYALURONIQUE OU DES FRAGMENTS DE CELUI-CI POUR LA PREPARATION D'UN MEDICAMENT POUR LA REGULATION DE LA DIFFERENCIATION HEMATOPOIETIQUE**
VERWENDUNG VON HYALURONSÄURE ODER FRAGMENTEN DAVON ZUR HERSTELLUNG EINES MEDIKAMENTES ZUR REGELUNG DER HÄMATOPOIETISCHEN DIFFERENZIERUNG
USE OF HYALURONIC ACID OR FRAGMENTS THEREOF FOR THE PREPARATION OF A MEDICAMENT FOR REGULATING HEMATOPOIETIC DIFFERENTIATION

(30) Priorité: 11.02.1999 FR 9901644
(43) Date de publication de la demande: 07.11.2001
(73) Titulaire: INSTITUT NATIONAL DE LA SANTE ET DE LA RECHERCHE MEDICALE (INSERM), 75654 Paris Cédex 13 (FR)
(72) Inventeur: SMADJA-JOFFE, Florence, F-92260 Fontenay aux Roses (FR); CHARRAD, Rachida-Sihem, F-94800 Villejuif (FR); CHOMIENNE, Christine, F-75007 Paris (FR); DELPECH, Bertrand, F-76410 Saint Aubin les Elbeuf (FR); JASMIN, Claude, F-94200 Charenton (FR)
(74) Mandataire: Armengaud Ainé, Alain
(86) Numéro de dépôt international: FR0000349
(87) Numéro de publication internationale: WO00047163

(56) Documents cités:
- EP-A- 0 240 098
- EP-A- 0 795 560
- DE-C- 19 802 540
- SMADJA-JOFFE F ET AL: "CD44 and hyaluronan binding by human myeloid cells." LEUKAEMIA AND LYMPHOMA, vol. 21, no. (5-6), 1996, pages 407-20, XP000856598 SWITZERLAND
- LI Y ET AL: "CD44: A signaling molecule for differentiation of HL60 myeloid leukemic cell line (Meeting abstract)." PROCEEDINGS OF THE AMERICAN ASSOCIATION FOR CANCER RESEARCH, vol. 36, mars 1995 (1995-03), page 215 XP000857230
- LI, Y ET AL: "The adhesion molecule CD44 mediates granulocytic differentiation of HL60 myeloid leukaemia cells and enhances the differentiation of CD34+ haematopoietic progenitors" BRITISH JOURNAL OF HAEMATOLOGY, vol. 93, no. 2, 1996, page 346 XP000949247
- MORIMOTO K C ET AL: "CD44 mediates hyaluronan binding by human myeloid KG1A and KG1 cells." PROCEEDINGS OF THE AMERICAN ASSOCIATION FOR CANCER RESEARCH ANNUAL MEETING, 1994, vol. 35, mars 1994 (1994-03), page 20 XP000857229
- DELPECH B ET AL: "Expression of the hyaluronan-binding glycoprotein hyaluronectin in leukemias." LEUKEMIA, FEB 1993, 7 (2) P172-6, vol. 7, no. 2, février 1993 (1993-02), pages 172-176, XP000856619 ENGLAND
- MCKEE CHARLOTTE M ET AL: "Hyaluronan (HA) fragments induce chemokine gene expression in alveolar macrophages: The role of HA size and CD44." JOURNAL OF CLINICAL INVESTIGATION, 1996, vol. 98, no. 10, 1996, pages 2403-2413, XP000856600
- GHAFFARI S ET AL: "Altered patterns of CD44 epitope expression in human chronic and acute myeloid leukemia." LEUKAEMIA, vol. 10, no. 11, 1996, pages 1773-1781, XP000856618 ENGLAND
- LEGRAS, S. ET AL: "CD44-mediated adhesiveness of human hematopoietic progenitors to hyaluronan is modulated by cytokines" BLOOD, vol. 89, 1997, pages 1905-1914, XP000946153
- CHARRAD RS ET AL: "Ligation of the CD44 adhesion molecule reverses blockage of differentiation in human acute myeloid leukemia" NATURE MEDICINE, vol. 5, no. 6, juin 1999 (1999-06), pages 669-676, XP000857226 UNITED STATES

## Description

La présente invention est, de manière générale, relative à des moyens permettant la régulation de la différenciation de cellules hématopoïétiques. De manière remarquable, les moyens de régulation selon l'invention s'appliquent à la différenciation de cellules dont la différenciation ne correspond plus à un profil normal et notamment à des cellules dont la différenciation est bloquée (cellules leucémiques, et en particulier blastes de leucémies aiguës myéloblastiques). Selon un autre aspect remarquable, les moyens de régulation selon l'invention s'appliquent également à la différenciation de cellules hématopoïétiques. Les moyens de régulation selon l'invention permettent en effet d'induire ou stimuler la différenciation de cellules leucémiques, et de blastes LAM en particulier, ainsi que celle de cellules souches selon la voie granulocytaire tout comme selon la voie monocytaire.

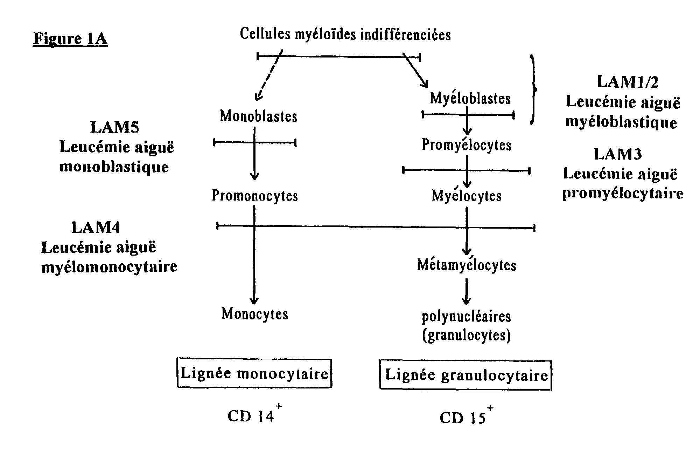

Différents types de leucémies peuvent être identifiés : les leucémies lymphoblastiques, qui comprennent notamment les leucémies aiguës lymphoblastiques (LAL) ou lymphomes, et les leucémies myéloblastiques qui comprennent notamment les leucémies aiguës myéloblastiques (LAM). Les LAM représentent environ la moitié des leucémies, soit environ 1000 nouveaux cas par an en France et 6500 aux USA, avec une incidence qui croît exponentiellement au-delà de 40 ans. Les LAM correspondent à un blocage de la différenciation des cellules myéloïdes à un stade immature, et se manifestent par l'envahissement de la moelle osseuse et du sang circulant par des cellules blastiques, dont les caractéristiques cytologiques définissent les différents sous-types de LAM classés MI à M7 (classification Française-Américaine-Britannique FAB), les plus fréquents étant les types MI à M5 *(cf* figure 1A).
Malgré des avancées thérapeutiques spectaculaires au cours de ces dernières années, les LAM restent une pathologie grave puisque la première rémission, bien qu'étant inductible dans 70% des cas, n'excède souvent pas la durée d'un an, et que 60% des patients rechutent dans les 5 ans. Les traitements des LAM en rechute, qui font largement appel à la greffe de moelle osseuse, connaissent d'importantes limitations en raison de la rareté des donneurs apparentés, et d'une limite d'âge de 45 ans.
Récemment, l'induction de la différenciation des blastes leucémiques en granulocytes matures par l'administration d'acide rétinoïque (AR, ou acide tout-*trans*rétinoïque ATRA) a spectacuairement amélioré l'évolution clinique des patients atteints de LAM M3, dont le taux de guérison atteint actuellement 70% à 5 ans. Ce traitement différenciateur n'est toutefois applicable qu'aux patients atteints de LAM de sous-type M3, un sous-type rare qui ne représente que 10% des LAM environ, et pose des problèmes de résistance *in vivo.* Ce traitement reste de plus inefficace pour les autres types de LAM.

La présente invention fournit quant à elle des moyens de régulation de la différenciation de cellules hématopoïétiques qui, de manière particulièrement remarquable, peuvent être appliqués aux cas de cellules dont la différenciation est bloquée, telles que des cellules leucémiques, et, de manière remarquable, des blastes LAM. Les moyens de régulation selon l'invention permettent en effet d'induire ou stimuler la différenciation de cellules leucémiques, et notamment de cellules LAM, et sont, de manière remarquable :
- efficaces sur des blastes leucémiques directement issus de patients (blastes LAM notamment) : ils sont en effet efficaces non pas simplement contre des lignées cellulaires modèles, c'est-à-dire des lignées d'étude conçues de manière à pouvoir auto-proliférer aisément *in vitro*, mais de manière remarquable, sont efficaces contre des cellules leucémiques directement issues de patients, c'est-à-dire des cellules qui sont peu ou pas capables de se diviser in *vitro*, et dont la survie dans de telles cultures est limitée dans le temps (moins d'une semaine en général), et
- efficaces contre non pas un seul mais plusieurs sous-types de LAM : ils permettent notamment d'induire la différenciation de blastes LAM M1/2, M3, M4, M5, qui sont les sous-types les plus fréquents. Il n'est de plus pas exclu qu'ils puissent être utilisés contre les sous-types moins fréquents de LAM, et les LAM6 et/ou LAM7 en particulier.
Les moyens de régulation selon l'invention sont donc efficaces contre différents sous-types de LAM, y compris contre les sous-types pour lesquels aucun traitement différenciateur efficace n'avait encore pu être réalisé (sous-types M1/2, M4, M5). En effet, ils permettent non seulement d'induire la différenciation selon la voie granulocytaire de blastes bloqués au stade M3, mais permettent également 1) de stimuler la différenciation selon les voies granulocytaire et monocytaire de blastes bloqués à un stade très immature (stade M1/M2) et de blastes bloqués au stade M4, et 2) d'induire la différenciation selon la voie monocytaire de blastes bloqués au stade M5.

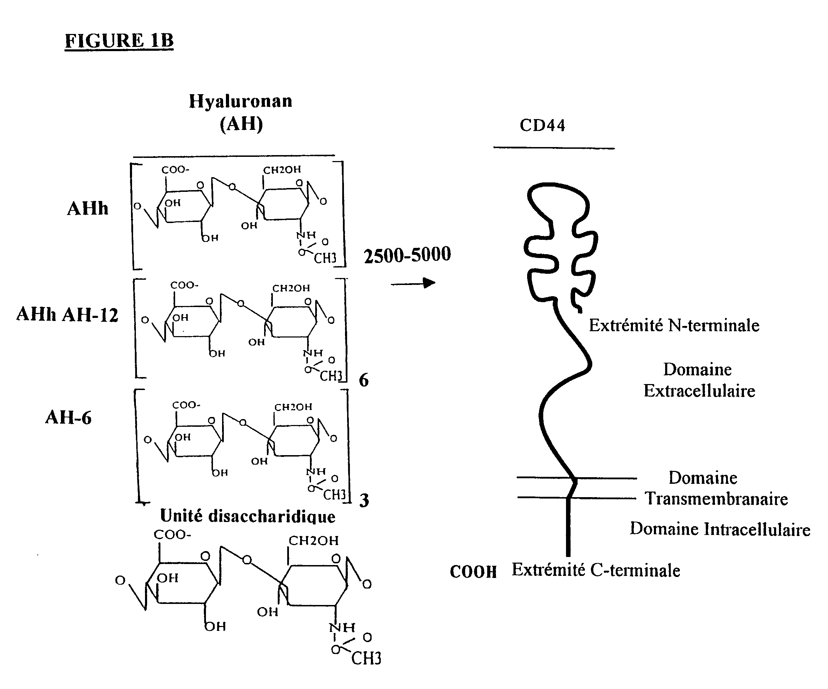

Avantageusement, les moyens de régulation selon l'invention permettent non seulement d'induire ou stimuler la différenciation de cellules leucémiques directement issues de patients, et de cellules LAM en particulier, mais peuvent également être utilisés pour inhiber la prolifération *in vivo* de telles cellules leucémiques.
Les moyens selon l'invention sont également efficaces pour réguler la différenciation de cellules hématopoïétiques normales très immatures (non complètement différenciées), et notamment celle de cellules souches ne présentant aucun antigène de différenciation telles que des cellules hématopoïétiques normales CD14⁻ CD15⁻ (cellules CD34⁺, voire CD34⁻). Ils permettent leur différenciation non seulement selon la voie monocytaire, mais aussi selon la voie granulocytaire, et sont efficaces sur des cellules issues de patients, et également *in vivo*.
Les moyens de régulation selon l'invention présentent également l'intérêt de n'être que peu ou pas susceptibles de toxicité pour le patient, jusqu'à des doses de plusieurs mg, ce qui constitue un grand avantage pour le patient, et ce qui permet l'utilisation du produit à des doses efficaces.
La présente invention vise ainsi l'utilisation pour la fabrication d'un médicament destiné à induire ou stimuler la différenciation de cellules choisies parmi le groupe constitué par des cellules leucémiques et des cellules souches CD14⁻ CD15⁻, caractérisé en ce qu'il comprend au moins un polymère comprenant une quantité efficace d'unités disaccharidiques composées chacune d'une molécule à structure de N-acétyl-D-glucosamine liée par liaison 0-glucosidique β1,4 à une molécule à structure d'acide glucuronique. Une représentation d'une telle unité disaccharidique est donnée à la figure 1B. Il peut être noté que le terme "polymère" couvre, dans la présente demande, aussi bien des oligomères que des polymères, et que les termes " médicament " et '' traitement " couvrent, dans la présente demande, toute forme de contrôle d'un état considéré pathologique ou inapproprié, y compris la thérapie, la prévention de l'aggravation de l'état pathologique, la palliation ou l'adoucissement des conditions de vie du patient. Avantageusement, l'utilisation dudit polymère selon l'invention permet la fabrication d'un médicament qui outre ses capacités à induire ou stimuler la différenciation de cellules leucémiques, peut être utilisé pour inhiber la prolifération de telles cellules leucémiques.
Par "quantité efficace", est entendu, dans la présente demande, un nombre d'unités disaccharidiques permettant au polymère résultant d'induire ou stimuler la différenciation des cellules leucémiques visées de manière significative. Des exemples de moyens permettant de tester si un polymère contient un nombre approprié d'unités disaccharidiques comprennent la mise en contact de ce polymère avec les cellules leucémiques visées, et notamment avec de telles cellules prélevées sur des humains, sous conditions physiologiques. Des exemples de telles mises en contact sont connus de l'homme du métier, certains en sont donnés dans la partie "exemples" ci-dessous. Brièvement, par conditions physiologiques, nous entendons, dans la présente demande, des conditions *in vivo*, ou bien des conditions *in vitro* mimant au mieux le *in vivo* (dans le cas d'un médicament destiné à l'homme, par exemple : milieu permettant la culture des cellules leucémiques visées tel que RPMI 1640/10% sérum (sérum de veau foetal SVF ou sérum autologue), température adaptée aux cultures cellulaires considérées, c'est-à-dire communément de l'ordre de 37°C environ, atmosphère saturée en humidité et contenant air et CO₂ en proportions adaptées aux cultures cellulaires considérées). Par cellules leucémiques prélevées sur des humains, nous entendons, dans la présente demande, des cellules fraîchement prélevées, et/ou des cellules ayant été conservées par congélation après prélèvement. De telles cellules leucémiques peuvent être notamment obtenues par prélèvement sanguin ou par prélèvement de cellules de la moelle osseuse, puis par récupération des globules blancs, avec éventuellement élimination des lymphocytes. Par cellules souches CD14⁻ CD15⁻, on entend en particulier des cellules hématopoïétiques normales capables de progénie et d'auto-réplication, et qui ne présentent aucun antigène de différenciation telles que CD14, CD15, c'est-à-dire des cellules souches CD14⁻, CD15- qui sont CD34⁺, voire CD34⁻.
Avantageusement, une quantité efficace desdites unités saccharidiques selon l'invention correspond à un nombre d'unités disaccharidiques supérieur ou égal à 3 environ. En dessous de 3 unités, l'efficacité de l'utilisation selon l'invention apparaît en effet beaucoup moins rentable industriellement. Un polymère répondant à de telles caractéristiques correspond notamment à de l'acide hyaluronique (AH), grosse molécule à 2500-5000 unités disaccharidiques (formule GlcAU(β1-3)-[GlcNAc(β1-4)GlcAU(β1-3)]ₙ-GIcNAc), ou à un fragment d'AH comprenant au moins trois unités disaccharidiques (à partir de AH-6). Préférentiellement, ladite quantité efficace correspond à un nombre d'unités disaccharidiques compris entre 3 et 10 (bornes incluses). Une utilisation préférée selon l'invention comprend donc l'utilisation pour la fabrication dudit médicament de fragments d'AH à au moins 3, et à au plus 10 unités disaccharidiques environ (de AH-6 à AH-20) : AH-6 et/ou AH-12, par exemple. Selon les effets recherchés, on dispose également de produits d'intérêt parmi les polymères comprenant plus de 10 desdites unités disaccharidiques. Notamment, les polymères présentant de 10 à 100 unités disaccharidiques sont également efficaces. L'utilisation de fragments présentant un petit nombre d'unités disaccharidiques est préférée pour de simples raisons de facilité de production. L'homme du métier pourra optimiser le choix d'un nombre d'unités disaccharidiques et le choix de quantités efficaces.
L'homme du métier dispose de plusieurs sources d'un polymère approprié à une utilisation selon l'invention : il peut par exemple être extrait de sources naturelles (pour AH : cordon ombilical humain, streptocoque, crête de coq, notamment), et éventuellement soumis à une digestion enzymatique *(cf.* partie "exemples" ci-dessous, digestion de AH par hyaluronidase), et/ou directement acheté auprès de fournisseurs tels que ICN Pharmaceuticals, Sigma (AH, fragments d'AH, par exemple). Ledit polymère peut également être utilisé à l'état de sel, tel que du hyaluronate de sodium ou de potassium sous forme de poudre, ou dissout en solution saline. Un tel polymère peut également présenter des modifications chimiques, de manière notamment à moduler la spécificité dudit polymère vis-à-vis des cellules leucémiques cibles (cellules LAM en particulier), à moduler sa durée de vie et/ou sa biodisponibilité.
Une utilisation selon l'invention peut notamment comprendre une utilisation dudit polymère pour la fabrication d'un médicament dont la dose unitaire est comprise entre 1 et 10 mg/kg, avantageusement entre 2 et 5 mg/kg, notamment de l'ordre de 3 mg/kg. Cette dose peut être augmentée ou diminuée (dose unitaire) et/ou répétée (dans le temps) pour optimiser l'efficacité du produit. Le polymère utilisé selon l'invention n'étant pas a priori toxique, sa posologie peut en effet être adaptée au patient considéré, en fonction par exemple des résultats de suivi de la maladie. L'invention fournit, à ce titre, une méthode *in vitro* qui permet de prédire, pour un patient donné, l'efficacité thérapeutique, en particulier anti-leucémique, d'un médicament obtenu par une utilisation selon l'invention. La méthode *in vitro* de prédiction selon l'invention comprend notamment :
- la mise en contact, en conditions physiologiques (par exemple, 37°C environ, milieu adapté à la culture cellulaire visée tel que RPMI 1640/10% sérum (sérum de veau foetal SVF ou sérum autologue), atmosphère saturée en humidité et contenant air et CO₂ en proportions adaptées), du médicament testé avec des cellules caractéristiques de l'état pathologique ou inapproprié considéré, issues du patient donné, et dans le cas d'un patient leucémique, avec des blastes leucémiques issus dudit patient,
- l'observation *in vitro* de l'existence ou de l'absence significative de l'effet thérapeutique désiré par rapport au témoin négatif, et dans le cas d'un patient leucémique, l'observation de l'existence ou de l'absence d'au moins un effet différenciateur significatif sur lesdites cellules par rapport au témoin négatif *(cf.* ci-dessous et dans la partie "exemples" pour des illustrations de tels effets),
- la prédiction d'une bonne efficacité thérapeutique (en particulier anti-leucémique) *in vivo* du médicament testé pour le patient considéré dans le cas où ledit lesdits effet(s) thérapeutique(s), en particulier différenciateur(s), est (sont) observés comme étant présent *in vitro.*
Un modèle expérimental animal peut également être utilisé.
De telles méthodes de prédiction selon l'invention constituent un bon outil pour adapter la posologie d'administration (dose unitaire, fréquence) d'un médicament selon l'invention.
Selon un mode variant de réalisation, une utilisation selon l'invention peut comprendre l'utilisation d'un agent mimétique dudit polymère (mimétique de l'acide hyaluronique ou d'un fragment de cet acide notamment), et d'un agent agoniste en particulier, tel qu'obtenu par criblage dans une banque chimique et/ou biologique. Des moyens de réaliser de tels criblages ou sélections sont connus de l'homme du métier : ils peuvent être notamment réalisées par criblages fonctionnels et/ou différentiels, par cytométrie de flux par exemple (sélection de composés capables de se lier aux mêmes cibles cellulaires que ledit polymère, et à CD44 notamment, et capables de produire au moins un effet différenciateur et/ou anti-prolifératif équivalent tout au moins en nature). Un tel agent mimétique ou agoniste peut être utilisé, selon l'invention, en alternative de l'utilisation dudit polymère ci-dessus présentée, ou bien en complément de cette utilisation. Sont avantageusement utilisés ceux de ces mimétiques ou agonistes qui ne sont pas toxiques pour l'homme, et/ou qui ne sont pas susceptibles d'induire des réactions antigéniques inappropriées. Ainsi, si la banque criblée est une banque d'anticorps (monoclonaux notamment), on pourra avantageusement choisir, pour la fabrication dudit médicament, des anticorps (monoclonaux) humains ou humanisés *(cf.* exemples).
Une utilisation selon l'invention peut également comprendre, outre ledit polymère ou mimétique, d'autres agents actifs pour l'induction et/ou la stimulation de la différenciation de cellules hématopoïétiques, et/ou de cellules leucémiques, et/ou de cellules LAM en particulier, tels que par exemple des cytokines. Ces autres agents actifs peuvent être incorporés audit médicament, ou être administrés parallèlement à ce médicament et présentés sous forme de kit comprenant d'une part ces autres agents, et d'autre part ledit polymère. Ledit polymère ou mimétique est donc, dans le médicament selon l'invention, un agent actif qui peut être utilisé à titre de co-agent actif

Ladite utilisation peut également comprendre, outre l'utilisation dudit polymère ou mimétique, l'utilisation d'un composé adjuvant capable de stimuler la liaison dudit polymère à sa cible cellulaire, tel qu'un anticorps anti-CD44 capable de stimuler une telle liaison, ou un fragment (Fab, (Fab')₂, Fv, CDR) d'un tel anticorps. Il peut être à ce sujet souligné que tout produit en général, et anticorps en particulier, considéré comne activateur d'une cible cellulaire telle que CD44 ne constitue pas obligatoirement un produit à activité différenciatrice et/ou anti-proliférative par liaison à sa cible et à CD44 en particulier, et que tous les produits (anticorps notamment) considérés comme différenciateurs et/ou anti-prolifératifs par liaison à une cible cellulaire telle que CD44 ne constituent pas nécessairement un produit capable de stimuler la liaison dudit polymère ou mimétique à cette cible : certains anticorps différenciateurs anti-CD44 s'opposent à, et inhibent la liaison de AH à CD44 *(cf.* partie "exemples"). L'homme du métier dispose néanmoins de moyens (cytométrie en flux par exemple) permettant de déterminer si un composé est ou non capable de stimuler, de manière satisfaisante pour les applications visées, la liaison dudit polymère ou mimétique à sa cible cellulaire, et à CD44 en particulier.
Une utilisation dudit polymère selon l'invention permet la fabrication dudit médicament sous toute formulation galénique adaptée à l'administration souhaitée, et notamment sous forme de comprimés, granules, capsules, forme pulvérulente, suspension, solution buvable, solution injectable, patch. Cette adaptabilité technique constitue un avantage notable de l'utilisation selon l'invention. Une utilisation dudit polymère selon l'invention permet la réalisation de solution, et notamment de solution saline. De telles fabrications sont réalisées sous des conditions adaptées aux propriétés physico-chimiques du polymère ou mimétique utilisé, et notamment à des conditions de pH, concentrations qui lui sont adaptées.
Une utilisation selon l'invention peut comprendre, outre l'utilisation dudit polymère ou mimétique, l'utilisation de tout composé ou excipient adapté à la formulation galénique souhaitée, et notamment tout véhicule pharmaceutiquement inerte approprié. Une solution injectable, notamment par voie intraveineuse, apparaît être une formulation d'intérêt aisément réalisable, ledit polymère étant facilement soluble en solution saline équilibrée. Cette solubilité représente un avantage par rapport à l'ATRA, qui n'est pas soluble en solution saline.
Avantageusement, une utilisation selon l'invention conduit à un médicament dont la mise en contact, en conditions physiologiques, avec un nombre statistiquement représentatif d'échantillons de cellules leucémiques (blastes) prélevées sur des humains se traduit, au niveau desdites cellules, par au moins un effet différenciateur significatif tel que l'induction ou la stimulation d'une réduction du nitro-bleu de tétrazolium, et/ou une expression accrue d'antigènes spécifiques de cellules hématopoïétiques en maturation telles que CD14 (voie monocytaire) ou CD15 (voie granulocytaire), et/ou l'induction ou la stimulation de caractéristiques cytologiques spécifiques de cellules hématopoïétiques en maturation (telles que diminution du rapport noyau/cytoplasme, diminution du nombre de nucléoles, condensation de la chromatine, segmentation du noyau, nombre restreint de granulations azurophiles, contours cytoplasmiques devenant irréguliers). D'autres effets différenciateurs significatifs comprennent un événement moléculaire marqueur d'une différenciation hématopoïétique tel que dégradation de l'oncoprotéine PML-RARα, et/ou une induction ou une stimulation de phosphorylations de tyrosines intracellulaires, et/ou une induction ou une stimulation d'au moins un messager de facteur de différenciation tel qu'une cytokine différenciatrice (par exemple, G-CSF, M-CSF). De manière avantageuse, la mise en contact en conditions physiologiques d'un médicament selon l'invention avec un nombre statistiquement représentatif d'échantillons de cellules leucémiques (blastes) prélevées sur des humains se traduit, en outre, au niveau desdites cellules, par une inhibition significative de leur prolifération. Des moyens pour réaliser de telles mises en contact, de telles conditions physiologiques, et de telles cellules leucémiques sont connus de l'homme du métier, des exemples ont été donnés ci-dessus et sont plus avant présentés dans la partie "exemples" ci-dessous. Par nombre statistiquement représentatif d'échantillons, nous entendons, dans la présente demande, un nombre d'échantillons permettant une analyse statistique valable des résultats, et notamment un nombre supérieur à environ 10 échantillons, par exemple de l'ordre de 20-30 échantillons environ. Par effet significatif, nous entendons un effet moyen statistiquement significatif par rapport aux témoins négatifs : par exemple, un effet qui ne soit pas significativement observé, en moyenne, dans les témoins négatifs, et qui soit significativement observé, en moyenne, dans au moins 75 % environ des échantillons de test.
Le rôle dudit polymère étant l'objet de l'utilisation selon l'invention est un rôle direct. Ledit polymère agit en effet par liaison à une molécule à la surface desdites cellules, qui agit alors comme un récepteur transducteur d'un signal pro-différenciateur et/ou anti-prolifératif. Ledit polymère est capable de présenter cette activité en l'absence de tout autre produit différenciateur. Il est par exemple capable d'exercer son action différenciatrice *in vitro* dans un milieu à base de sérum (sérum de veau foetal (SVF) ou sérum autologue) sans adjonction de cytokine. En présence de cellules hématopoïétiques normales, telles que des cellules CD34⁺ progénitrices, il est capable d'exercer son action différenciatrice *in vitro* dans un milieu sans sérum. Des moyens d'observer de telles capacités sont connus de l'homme du métier. Des exemples en sont donnés dans la partie "exemples" ci-dessous.
Cette activité dudit polymère sur les cellules hématopoïétiques s'exerce notamment par activation du récepteur CD44. Il n'est pas exclu qu'elle puisse s'exercer (de manière indépendante ou conjointe) par l'intermédiaire de tout autre récepteur membranaire capable de fixer ledit polymère (AH, fragment d'AH, par exemple) et d'induire un signal différenciateur à la cellule exprimant ce récepteur. Un tel récepteur candidat peut être avantageusement choisi parmi les molécules de la famille des hyaladhérines (RHAMM, hyaluroncctine). L'homme du métier dispose de nombreux moyens permettant de tester si un récepteur candidat peut être reconnu par ledit polymère, et si ce récepteur transduit alors un signal de différenciation cellulaire. De tels moyens comprennent notamment :
i. la recherche, à l'aide de techniques de cytométrie en flux, par exemple, d'une liaison dudit polymère (AH et/ou fragments d'AH à au moins 3 unités disaccharidiques, notamment) à des cellules exprimant ledit récepteur candidat et d'une absence de liaison de ce(s) même(s) polymère(s) à ces mêmes cellules lorsque l'accès audit récepteur candidat est spécifiquement bloqué, et/ou
ii. la recherche d'au moins un effet différenciateur sur des cellules exprimant le récepteur candidat mises en présence dudit polymère (AH et/ou fragments d'AH à au moins 3 unités disaccharidiques, notamment), par comparaison avec le même type de cellules placées dans des conditions équivalentes mais en l'absence de ce(s) même(s) polymère(s). Des exemples de tels effets différenciateurs et/ou anti-prolifératifs peuvent être notamment trouvés ci-dessous dans la partie "exemples".
Afin, en revanche, d'éviter l'éventuelle liaison inappropriée dudit polymère à des molécules à la surface de cellules non visées, par exemple l'éventuelle liaison dudit polymère (AH et/ou fragments) au récepteur ICAM1 au niveau des sinus hépatiques, une utilisation selon l'invention peut en outre comprendre l'utilisation de composés bloquant lesdites molécules non visées, par exemple, des composés anti-ICAM1 bloquant la liaison dudit polymère à ICAM1, tels que des anticorps monoclonaux anti-ICAM1, ou de la chondroïtine sulfate qui en se liant à ICAM1 bloque l'accessibilité de ICAM1 à AH. Selon un autre mode de réalisation de l'invention, il peut être en outre utilisé un composé capable de prévenir la liaison à une cible cellulaire inappropriée dudit polymère ou molécule mimétique. Des exemples de tels composés comprennent des anticorps monoclonaux anti-ICAM1, ou un fragment (Fab, (Fab')₂ Fv, CDR) de tels anticorps.
Selon une disposition avantageuse de l'invention, lesdites cellules leucémiques sont des cellules de leucémie myéloblastiques (blastes), et des cellules de leucémie aiguë myéloblastique en particulier. Il peut notamment s'agir de blastes LAM1/2 et/ou LAM3 et/ou LAM4 et/ou LAM5. L'utilisation selon l'invention est en effet avantageusement destinée à la fabrication d'un médicament anti-leucémie myéloblastique, et anti-LAM1/2 et/ou anti-LAM3 et/ou anti-LAM4 et/ou anti-LAM5 et/ou anti-LAM6 et/ou anti-LAM7 en particulier.
Comme ci-dessus précisé, et comme illustré dans les exemples, l'utilisation dudit polymère selon l'invention permet donc la fabrication d'un médicament qui est destiné à stimuler ou induire la différenciation de cellules dont la différenciation est bloquée, en particulier des cellules leucémiques, et de manière remarquable, des blastes LAM. Ledit médicament ainsi obtenu selon l'invention n'en est pas moins capable, en conditions physiologiques, de stimuler ou induire la différenciation de cellules souches hématopoïétiques normales (non complètement différenciées), sans inhiber leur prolifération : il peut en effet stimuler ou induire la différenciation de cellules souches hématopoïétiques humaines saines (CD14⁻ CD15⁻ CD34⁺, voire CD34⁻). Par conséquent, l'utilisation selon l'invention permet ainsi également la fabrication d'un médicament qui peut être administré à des patients diagnostiqués leucémiques afin de stimuler ou induire la différenciation de leurs cellules souches humaines normales. Elle permet de même la fabrication d'un médicament qui peut être administré à des patients non leucémiques afin de stimuler ou induire la différenciation de leurs cellules hématopoïétiques normales (non complètement différenciées), ceci notamment dans un objectif de traitement d'une aplasie, ou d'une neutropénie. La présente invention a donc pour objet, de manière générale, l'utilisation d'un polymère comprenant une quantité efficace d'unités disaccharidiques composées chacune d'une molécule à structure de N-acétyl-D-glucosamine liée par liaison O-glucosidique β 1,4 à une molécule à structure d'acide glucuronique part la fabrication d'un médicament destiné à induire ou stimuler la différenciation de cellules souches CD14⁻ CD15⁻ ou de cellules leucémiques, et de blastes LAM en particulier.

La présente invention est illustrée par les exemples suivants. La présente invention comprend également toute variante de mode de réalisation que l'homme du métier peut mettre en oeuvre, sans expérimentation indue, à partir de l'enseignement donné par la présente demande (description, exemples, revendications et figures inclus) et des moyens de l'art antérieur.
Dans la partie "exemples" qui suit, il est fait référence aux figures suivantes :
- sur la figure 1A, les plus fréquents sous-types de LAM (classification FAB : LAM M1/M2 ou LAM1/2 , LAM M3 ou LAM3 ; LAM M4 ou LAM4 ; LAM M5 ou LAM5) sont indiqués au niveau du stade de différenciation myéloïde au blocage duquel ils correspondent,
- la figure 1B donne une représentation schématique de la molécule de surface cellulaire CD44, et de molécules d'acide hyaluronique (AH), qui sont capables de se lier à CD44 : acide hyaluronique humain AHh à 2500-5000 unités disaccharidiques, fragment d'AHh à 6 unités disaccharidiques (AH-12) et fragment d'AHh à 3 unités disaccharidiques (AH-6),
- les figures 2A et 2B illustrent le fait que l'acide hyaluronique (AH) est capable d'induire la différenciation de tous les sous-types des blastes LAM :

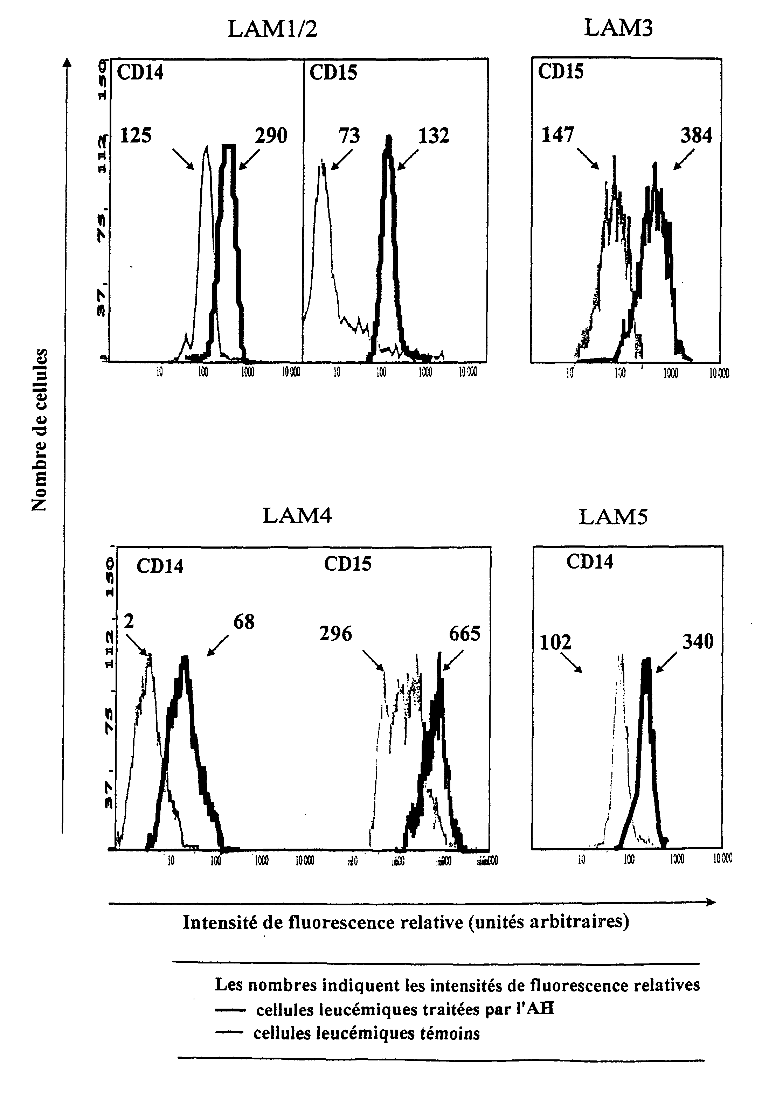

   - figure 2A : graphes représentant le nombre de cellules CD14⁺ et CD15⁺ induites par utilisation d'AH sur des LAM1/2, 3, 4 et 5, en fonction de l'intensité de fluorescence (courbes noires : utilisation d'AH ; courbes grises plus à gauche : témoins négatifs),

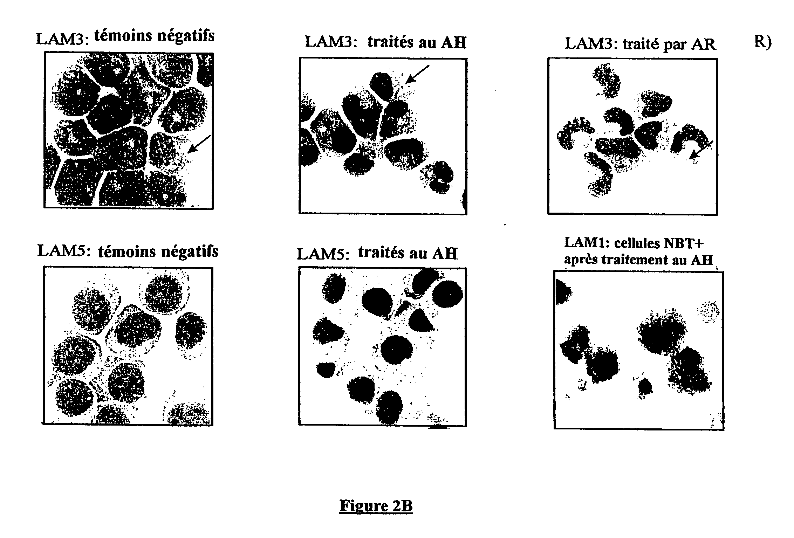

   - figure 2B : clichés illustrant l'induction sur des blastes LAM par AH (activation de CD44) de caractéristiques cytologiques spécifiques de cellules matures,

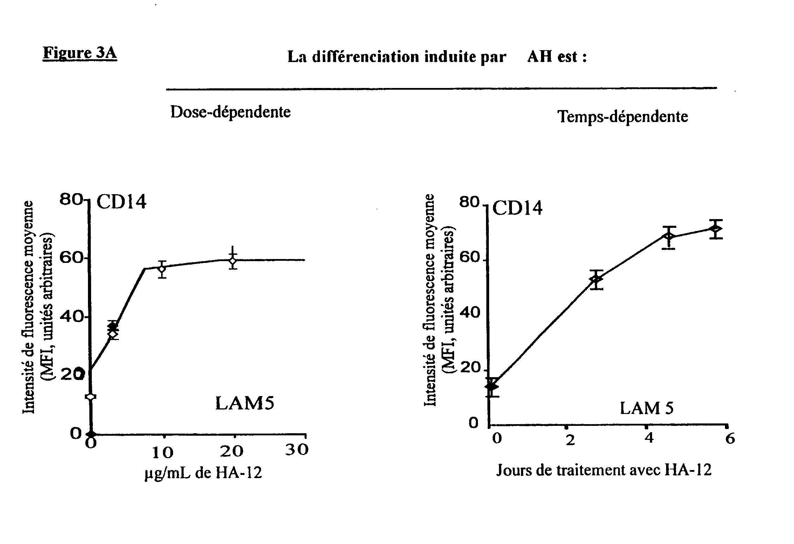

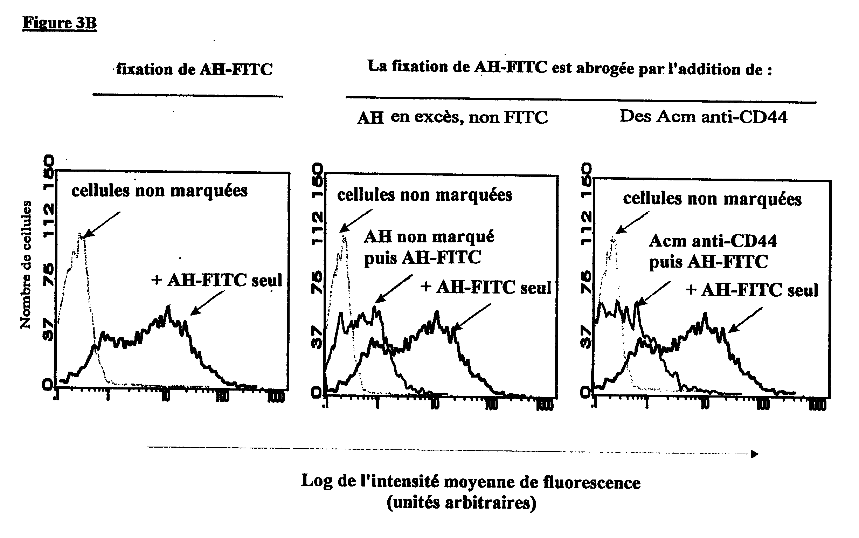

- les figures 3A et 3B illustrent le caractère dose-dépendant et temps-dépendant de la différenciation induite par l'acide hyaluronique (AH) :
   - figure 3A : intensité moyenne de fluorescence (MFI CD14) mesurée sur des blastes LAM5 en fonction de la dose de (µg/ml) AH-12 (graphe de gauche), et en fonction du temps d'incubation en présence de AH-12 (graphe de droite),
   - figure 3B : liaison de AH-FITC et inhibition de cette liaison, telle qu'illustrée par le nombre de cellules en fonction du log de l'intensité de fluorescence pour (courbes identifiées de gauche à droite pour chaque graphe) :
      - graphe de gauche : des cellules non marquées, et des blastes LAM incubés avec AH-FITC seul,
      - graphe central : cellules non marquées, blastes LAM incubés avec AH non marqué puis avec AH-FITC, blastes LAM incubés par AH-FITC seul,
      - graphe de droite : cellules non marquées, blastes LAM incubés avec des anticorps monoclonaux (Acm) anti-CD44, puis avec AH-FITC, blastes LAM incubés avec AH-FITC seul,

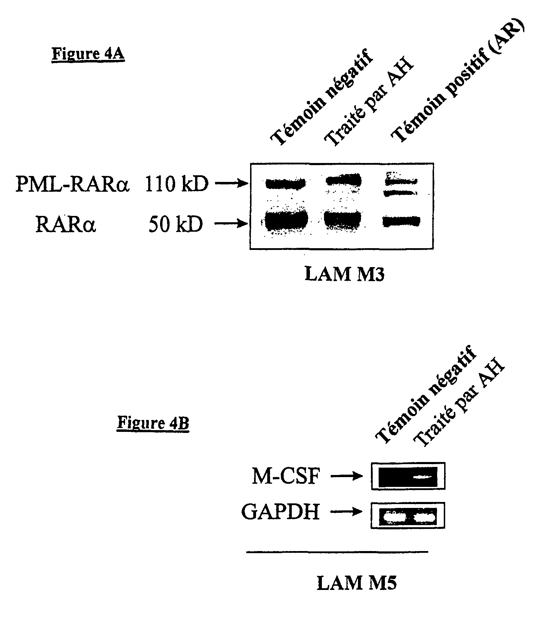

- les figures 4A, 4B, 4C illustrent des événements moléculaires marquant l'induction par AH (activation de CD44) de la différenciation de blastes LAM :
   - figure 4A : dégradation de l'oncoprotéine PML-RARα (bandes du haut à 110 kDa) chez des blastes LAM M3, 24 heures après traitement avec AH, et maintien de la protéine sauvage RARα (bandes du bas à environ 50kD), telles que détectées à l'aide du système chimioluminescent ECL (piste 1 : témoin négatif, piste 2 : blastes traités au AH, piste 3 : témoin positif (blastes traités au AR)),
   - figure 4B : induction par AH de la synthèse de transcrits M-CSF chez des blastes LAM M5 (deux photos groupées : M-CSF en haut de ce groupe, marqueur GAPDH en bas), telle que visualisée par électrophorèse de l'ARN total sur gel d'agarose et hybridations spécifiques (pour chaque gel : piste 1 = témoin négatif, piste 2 : blastes traités au AH),

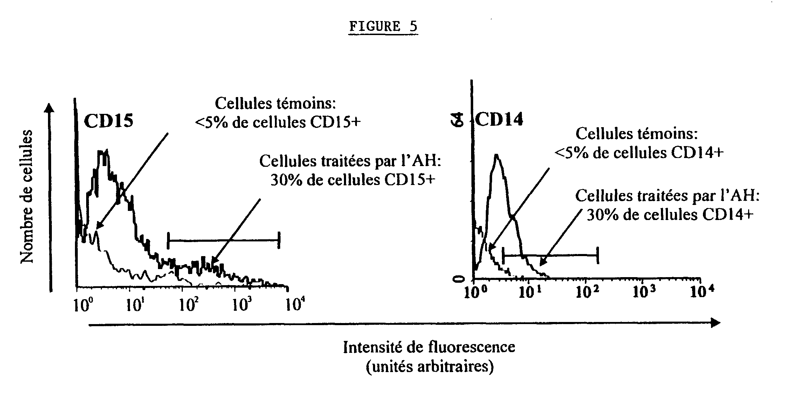

   - Figure 5 : induction par des fragments d'AH de la différenciation de cellules hématopoïétiques normales très immatures (cellules souches CD34⁺ CD14⁻ CD15⁻) selon la voie monocytaire et selon la voie granulocytaire.

### EXEMPLE 1 : induction, par AH et via notamment CD44, de la différenciation de blastes LAM

### Matériels et Méthodes

**Patients LAM.** Des échantillons de sang périphérique ou de moelle osseuse leucémique ont été prélevés au moment du diagnostic, après consentement informé, sur 36 patients atteints de leucémie aiguë myéloïde (LAM). Le diagnostic de la maladie et sa classification répondent aux critères de la classification Française-Américaine-Britannique (FAB). Tous les patients ont présenté plus de 60% de blastes dans le sang périphérique.

**Séparation des blastes LAM.** Des cellules LAM fraîches ou congelées ont été enrichies par centrifugation selon le gradient de densité en présence de Ficoll, et lavées dans du milieu RPMI 1640 contenant 10% de sérum de veau foetal (SVF). Les cellules congelées ont été décongelées à température ambiante dans du milieu RPMI 1640 contenant 50% de SVF, puis lavées deux fois dans du milieu RMPI 1640 additionnées de SVF à 10%. On a éliminé de tous les échantillons les lymphocytes B et T, ainsi que les monocytes dans le cas des échantillons LAM1/2 et LAM3. Cette élimination a été effectuée par immunoadsorption spécifique des billes Dynabeads (Dynal, Oslo, Norvège) couvertes d'anticorps monoclonaux dirigés contre les antigènes de surface spécifiques CD2 et CD19 (lymphocytes) et CD14 (monocytes), en suivant les instructions du fabricant. Sont ainsi obtenues des suspensions cellulaires contenant plus de 95% de blastes LAM.

### Anticorps monoclonaux (Acm) anti-CD44.

Différents Acm anti-CD44 ont été utilisés dans les tests d'induction d'une différenciation : F1O-44-2 (IgG2a, Serotec, Kidlington Oxford, UK), et Hermes-1 (IgG2a, hybridome disponible auprès de Developmental Studies Hybridoma Bank, Iowa), notamment. Ces Acm sont tous deux capables de transmettre un signal activateur. Pour les témoins négatifs, ces Acm ont été remplacés par les IgG murines (non anti-CD44) du même isotype.
Différents anticorps monoclonaux anti-CD44 ont été utilisés pour les tests de liaison AH-FITC à CD44: notamment, l'Acm J173 (Coulter-Immunotech, Marseille-Luminy, France).
L'Acm 6D12 (IgG1, Coulter-Immunotech) a été utilisé dans les tests de phosphorylation de protéines sur résidus tyrosine.

### Acide Hyaluronique (AH) et témoins.

De l'acide hyaluronique issu de cordon ombilical humain (AHh, ref. 362421) a été obtenu auprès de ICN Pharmaceuticals (Costa Mesa, CA), dissout à 5 mg/ml dans de l'eau distillée et bouilli pendant 10 minutes. Plusieurs formes moléculaires de l'acide hyaluronique (AH) ont été utilisées : acide hyaluronique humain AHh, (forme à haute masse moléculaire (500 à 2000 kDa), et deux types de fragments oligosaccharidiques AH-6 et AH-12, obtenus après digestion de AHh par la hyaluronidase (Sigma, St Louis, MO), à 37°C pendant 6 heures, et isolement selon les techniques classiques sur une colonne de chromatographie sur gel AcA202 (Biosepara, Villeneuve La Garenne, France). AHh, AH-6 et AH-12 sont composés de 2.10³-10⁴, 6 et 12 unités saccharidiques respectivement. Toutes les préparations d'acide hyaluronique utilisées sont dépourvues d'endotoxine. La figure 1B donne une représentation schématique de la molécule d'acide hyaluronique humain (AHh, 2500-5000 unités disaccharidiques), fragment d'AHh à 6 unités disaccharidiques (AH-12), fragment d'AHh à 3 unités disaccharidiques (AH-6), et une représentation schématique de la molécule de surface cellulaire CD44, à laquelle AH est capable de se lier. Chaque unité disaccharidique est composée d'une molécule d'acide D-glucuronique liée à une molécule de N-acétyl-D-glucosamine. L'acide hyaluronique se lie à la molécule CD44 au niveau d'une région située à l'extrémité N-terminale du domaine extracellulaire.
A titre de témoins négatifs, les blastes LAM ont été cultivés en présence de sulfate de chondroïtine (Sigma), une glycosaminoglycanne sulfatée avec une structure proche de celle de l'acide hyaluronique et qui est susceptible de se lier à CD44. De plus, des blastes leucémiques issus de LAM3 ont été traités à l'acide tout-*trans*rétinoïque (AR) à titre de témoins positifs de différenciation pour ce sous-type de LAM.

**Traitement des blastes LAM avec AH.** Des suspensions cellulaires contenant plus de 95% de blastes LAM ont été déposées en triplicata à raison de 2.10⁵ cellules par ml de RPMI 1640/SVF 10% dans des plaques de culture tissulaire (Costar Corp., Cambridge, MA) à 96 puits contenant chacun 150 µl de milieu, et placées en incubation pendant 5 jours en présence de 20 µg/ml d'acide hyaluronique (AHh, AH-6 ou AH-12). L'AH a été ajouté aux concentrations spécifiées, et le sulfate de chondroïtine a été ajouté dans les témoins négatifs. Les plaques ont été placées dans un incubateur à 37°C sous atmosphère humide pendant 6 jours, et les cellules ont été soumises aux études de différenciation comme décrit ci-après.

### Evaluation de la différenciation myéloide

La différenciation a été recherchée en analysant 3 critères : 1) la capacité à effectuer une réaction d'oxydo-réduction en réponse à un ester de phorbol : cette réaction d'oxydo-réduction est détectée par la réduction du bleu de nitrotétrazolitun NBT, 2) l'expression d'antigènes spécifiques de la différenciation (CD14 spécifique des monocytes, et CD15 spécifique des granulocytes), et 3) des changements cytologiques spécifiques. Tous ces critères sont spécifiques de cellules granulocytaires ou monocytaires différenciées normales.
***Test* de réduction *au bleu de nitrotetrazolium (NBT) :*** La capacité à réduire le NBT a été mesurée selon les techniques classiques. Brièvement, 2.10⁵ cellules ont été mises en suspension dans 900 µl de milieu RPMI 1640, et ont été incubées en présence de 0,2 µg/ml de 12-O-tétradécanoylephorbol-13-acétate (TPA, Sigma) et de 0,5 mg/ml de NBT (Sigma) pendant 30 minutes à 37°C. La réaction a été arrêtée à 4°C, les cellules ont été cytocentrifugées et soumises au colorant de May-Grünwald-Giemsa. Le pourcentage de cellules contenant des dépôts noirs de réduction du NBT a été déterminé en duplicata, sous microscope optique, après examen de 300 cellules.

***Analyse par cytométrie en flux de l'expression de* CD14 *et* CD15 :** les blastes LAM ont été mis en suspension, à raison de 10⁵ blastes/ml de milieu de RPMI 1640 contenant 0,02% d'albumine de sérum bovin et 0,02 % de NaN₃ à 10⁵ cellules par ml , ils ont ensuite été incubés à 4°C pendant 30 minutes en présence d'Acm conjugués à de l'isothiocyanate de fluorescéine (FITC), et dirigés contre CD14 (IgG2b 5 µg/ml, Coulter Immunology, Hialeah, FL) ou dirigés contre CD15 (IgM 1 µg/ml, Becton Dickinson, San José, CA). Les Acm ont été utilisés aux concentrations de saturation. Les IgM et IgG2b murins conjugués à FITC proviennent de Coulter-Imnunotech (Coulter-Immunotech, Inc. Westbrook, Maine), et ont été utilisés dilués au 1:50. La liaison des Acm dirigés contre CD14 et CD15 a été quantifiée en mesurant, par cytométrie de flux, la fluorescence cellulaire relative à celle de cellules marquées par des IgG-FITC. La mesure a été effectuée en utilisant un FACSvantage (Becton Dickinson) équipé d'un laser à ions argon INNOVA70-4 (Coherent Radiation, Palo Alto, CA) réglé sur 488 nm et fonctionnant à 500 mW. Le cytomètre en flux a été calibré à l'aide de billes fluorescentes (Becton Dickinson). Chaque mesure a été réalisée sur 3000 cellules.
***Etude cytologique* :** Les frottis cellulaires, préparés en triplicata, ont été colorés par la coloration de May-Grünwald-Giemsa, et leur cytologie a été examinée sous microscope optique.

**Analyse de la dégradation de l'oncoprotéine PML-RARα :** Les protéines cellulaires totales ont été extraites des blastes traités et témoins, séparées sur gel de 8% d'acrylamide dans du dodécyl sulfate de sodium (SDS), et électrotransférées sur une membrane de nitrocellulose (Laboratoires BioRad). Après blocage des sites de fixation non spécifiques avec du lait écrémé à 5% dans du tampon de phosphate salin (PBS), les transferts ont été incubés une nuit durant, en présence d'une dilution au 1:2000 d'un anticorps de lapin polyclonal anti-RARα (Blood 88: 2826-2832, 1996 Raelson *et al.).* Après trois lavages pendant 20 minutes dans du PBS, la fixation de l'Ac polyclonal anti-RARα a été révélée par incubation avec un anticorps de chèvre anti-lapin marqué à la peroxydase, puis par chimioluminescence (système de détection ECL, Amersham Life Science, Arlington Heights, IL).

### Analyse de la phosphorylation des protéines sur résidus tyrosine

***Inducfion de phosphorylation de protéine sur résidus tyrosine :*** AH-12 (50 µg/ml) a été ajouté au temps t = 0 à 2.10⁵ blastes LAM mis en suspension, à température ambiante, dans 200 µl de milieu RPMI 1640 contenant 10% de SVF. A t = 1 min, 5 min, 15 min et 30 min, 200 µl de Permeafix Ortho (Coulter-Immunotech, Inc. Westbrook, Maine) ont été ajoutés pour arrêter les phosphorylations et perméabiliser les cellules. Après 40 minutes d'incubation à température ambiante, et trois lavages dans du PBS, les protéines phosphorylées au niveau des résidus tyrosine ont été marquées de manière spécifique avec l'Acm 6D12 (utilisé à 2 µg/ml) conjugué au FITC, et l'intensité du marquage a été mesurée par cytométrie en flux par référence au témoin isotypique (cellules marquées par IgG1 conjugué au FITC), comme décrit ci-dessus.
***Inhibition des phosphorylations de tyrosine par la génistéine*** *:* 2.10⁵ blastes LAM, mis en suspension dans 200 µl de milieu RPMI 1640/SVF à 10%, ont été incubés en présence de 50 nM/l de génistéine (Calbiochem-Novabiochem, San Diego, CA) pendant 1 heure à 37°C, puis en présence de AH pendant soit une heure (pour les études relatives à l'expression des transcrits de cytokine), soit cinq jours (pour les études relatives à la différenciation). Le test d'exclusion du bleu de Trypan a montré que les traitements ne sont pas cytotoxiques, la viabilité des cellules étant supérieure à 95%.
**Liaison de AH-FITC :** Une préparation de AH-FITC a été réalisée avec de l'AHh (acide hyaluronique humain) et du FITC selon les techniques classiques. Les cellules ont été lavées trois fois dans du tampon de phosphate salin (PBS), incubées avec AH-FITC à 2,5 µg/ml dans du PBS pendant 30 minutes sur de la glace, et lavées dans du PBS contenant du SVF à 2% et de l'azide de sodium à 0,02 % (Milieu de Marquage, La liaison de AH-FITC a été mesurée par cytométrie de flux, comme décrit ci-dessus, par référence à des cellules non marquées. Pour s'assurer que le marquage observé est spécifique d'AH, les cellules ont été préincubées à +4°C avec AH non fluorescent (100 µg/ml) et l'abrogation de la fixation de AH-FITC a été recherchée. L'implication du CD44 dans la fixation de AH-FITC a été démontrée en recherchant si des Acm anti-CD44 tels que notamment J173 (25 µg/ml) inhibent cette fixation.
**Etude par RT-PCR de l'expression des transcrits de cytokines :** l'ARN total a été extrait de 5.10⁵ cellules à l'aide du réactif de Trizol (Life Technologies, Cergy Pontoise, France), suivie d'une extraction au phénolchloroforme et d'une précipitation à l'isopropanol. Un microgramme d'ARN total chauffé à 70°C pendant 10 minutes a été utilisé comme matrice pour la synthèse du premier brin d'ADN complémentaire (ADNc), par addition de transcriptase inverse et d'hexamères aléatoires (Life Technologies). Le transcrit du gène de ménage de la glycéraldéhyde phosphodéshydrogénase (GAPDH) a été utilisé comme marqueur interne de la réaction PCR (produit d'amplification 0,24kb). Des quantités équilibrées d'ADNc ont été utilisées pour l'amplification par PCR des transcrits de cytokine, les amorces utilisées pour la PCR ont été les suivantes :
Facteur Stimulant la formation de Colonies de Macrophages
   (Macrophage Colony Stimulating Factor) : M-CSF : 5'-CATGACAAGGCCTGCGTCCGA-3' (SEQ ID N°1) et

5'-GCCGCCTCCACCTGTAGAACA-3' (SEQ ID N°2);

Facteur Stimulant la formation de Colonies Granulocytaires
(Granulocytic Colony Stimulating factor) : 
G-CSF : 5'-TTGGACACACTGCAGCTGGACGTCGCCGACTTT-3- (SEQ
ID N°3) et 5'-ATTGCAGAGCCAGGGCTGGGGAGCAGTCATAGT-3'
(SEQ ID N°4) (Genset, Ivry, France).
 Le protocole PCR a consisté en 30 cycles de 94°C pendant 1 minute, 58°C pendant 1 minute et 72°C pendant 1 minute, en utilisant un thermocycleur (Perkin Elmercetus, Norwalk, CT). Pour chaque expérience, deux témoins négatifs ont été soumis à l'ensemble des étapes. Les produits d'amplification par PCR (M-CSF : 395 pb, G-CSF : 470 pb) ont été séparés par électroporèse sur gel de 1% d'agarose, et visualisés par coloration au bromure d'éthidium. Afin de démontrer la spécificité de l'amplification, les produits d'amplification PCR ont été transférés sur une membrane Immobilon-S (Millipore Inc, France) et hybridés à des sondes oligonucléotidiques spécifiques marquées à l'extrémité 5' par du ³²P :

M-CSF 5'- TCAGCAAGAACTGCAACAACAGC-3' (SEQ ID N°5) ;
G-CSF 5'-GTGAGGAAGATCCAGGGCGA-3' (SEQ ID N°6) (Genset,
Ivry, France).

### Résultats et Discussions

### INDUCTION PAR AH D'UNE DIFFERENCIATION DES BLASTES LEUCEMIQUES DE LAM

Des blastes leucémiques ont été isolés du sang ou de la moelle osseuse de patients présentant différents sous-types de LAM (n = 24, Tableau I), et cultivés en présence d'acide hyaluronique (AH) pendant 5 jours *(cf.* matériels et méthodes). Les résultats obtenus sont résumés dans le tableau I ci-dessous.

**Tableau I :**

| **Différenciation de blastes LAM induite par AH** | | |
|---|---|---|
| Sous-type de LAM | Nombre de cas analysés | Nombre de cas de différenciation |
| LAM1/2 | 7 | 5 |
| LAM3 | 16 | 12 |
| LAM4 | 4 | 3 |
| LAM5 | 8 | 6 |
| Nombre total | 35 | 26 soit 74% |

Les sous types de LAM sont définis par les critères de la classification Française-Américaine-Britannique (FAB). La figure 1A indique schématiquement, pour les sous-types de LAM les plus fréquents (LAM1/2, LAM3, LAM4, LAM5), le stade de différenciation myéloïde au blocage duquel chaque sous-type correspond.

Les résultats obtenus montrent que l'AH stimule la différenciation de blastes leucémiques dans tous les sous-types de LAM (5/7 des LAM 1/2, 12/16 des LAM3, 3/4 des LAM4 et 6/8 des LAM5). De plus, l'étendue de cette différenciation mesurée par le test NBT a été aussi élevée dans des LAM3 et
LAM5 que celle atteinte après traitement des LAM3 par l'AR (acide tout-*trans*rétinoïque).
La figure 1B donne une représentation schématique de molécules d'acide hyaluronique (AH) : acide hyaluronique humain AHh à 2500-5000 unités disaccharidiques, fragment d'AHh à 6 unités disaccharidiques (AH-12) et fragment d'AHh à 3 unités disaccharidiques (AH-6). De manière notable, l'acide hyaluronique constitue dans le compartiment hématopoïétique un ligand de la molécule de surface cellulaire CD44.
Ces résultats de différenciation des blastes LAM ont notamment été visualisés par *(cf.* matériels et méthodes) :
- l'induction d'une capacité à réduire le nitro-bleu de tétrazolium,
- une expression accrue d'antigènes spécifiques de lignées, à savoir CD 15 spécifique de la différenciation granulocytaire et CD 14 spécifique de la différenciation monocytaire,
- l'induction de caractéristiques cytologiques spécifiques.
Premièrement, la capacité à produire une réaction d'oxydo-réduction a été analysée en utilisant le test **de réduction du nitro-bleu de tétrazolium (NBT**^{**+**}**).** Le tableau II ci-dessous illustre les pourcentages de blastes NBT positifs observés parmi des blastes issus de patients LAM1/2, LAM3, LAM4, LAM5, après traitement au AH ou après traitement témoin négatif comme décrit en matériels et méthodes (pour les LAM3 : témoin positif traité à l'AR).

**Tableau II :**

| **Induction de la capacité à réduire le nitro-bleu de tétrazolium** | | |
|---|---|---|
| **Type de LAM** | | **% cellules NBT**^{**+**} **(valeurs extrêmes)** |
| | témoins négatifs | traitées par AH |
| M1/M2 | <5 | 7-23 |
| M3 | <5 | 20%-80% (AR : 47-90) |
| M4 | 10%-38% | 43%-90% |
| M5 | <5 | 32%-70% |

Comme attendu dans les groupes de témoins négatifs, moins de 5% des blastes issus de LAM1/2, LAM3 et LAM5 sont observés NBT⁺. Par contraste, après incubation en présence d'AH *(cf.* matériels et méthodes), la proportion de blastes NBT⁺ a augmenté de manière significative pour tous les sous-types, ce qui indique qu'ils se différencient. En effet, dans 12 cas de LAM3 sur 16, le % de cellules NBT⁺ est compris entre 20% jusqu'à 80% (valeur médiane 42%). De plus, parmi 6 d'entre-eux, le % observé est aussi élevé que celui obtenu par traitement à l'acide tout-*trans*rétinoïque (plus de 50 % de cellules sont NBT⁺). De manière similaire, dans 6 cas de LAM5 sur 8, le % de cellules NBT⁺ est compris entre 32% et 70% (valeur médiane 52%). Dans 5 cas de LAM1/2 sur 7, le % de cellules NBT⁺ a augmenté jusqu'à atteindre 20-25% (valeur médiane 22%). Cette valeur est supérieure de manière significative à celle observée dans les témoins négatifs, mais plus faible que celles observées dans les cas de LAM3 et LAM5, ce qui indique que la maturation de ces blastes particulièrement immatures (LAM1/2) a été, aux doses et aux temps ici appliqués, plus limitée que dans les cas de LAM3 et LAM5. Dans le cas de LAM4 qui sont des cas de blastes plus matures, 10% à 38% (valeur médiane 18%) des témoins négatifs ont été observés NBT⁺. Après traitement, cette proportion augmente pour atteindre 43% à 90% (valeur médiane 55%, c'est-à-dire 3 fois plus haut que dans les témoins). Ces résultats indiquent que les molécules activatrices de CD44 telles que l'acide hyaluronique, ou ses fragments jusqu'à AH-6, provoquent la différenciation des blastes LAM parmi tous les sous-types.
Deuxièmement, nous avons mesuré par cytométrie de flux **le niveau d'expression d'antigènes spécifiques de lignées** sur des blastes LAM.
L'expression de CD15 a été utilisée pour suivre la différenciation de blastes LAM3 (sous-type promyélocytaire) parce qu'elle est spécifique de la lignée granulocytaire.

**Tableau III:**

| Augmentation du pourcentage de cellules exprimant les antigènes de différenciation CD14 (monocytaire) et/ou CD15 (granulocytaire) | | | | |
|---|---|---|---|---|
| Type de LAM | % cellules CD14⁺ valeurs médianes (valeurs extrêmes) | | % cellules CD15⁺ valeurs médianes (valeurs extrêmes) | |
| | témoins | traitées par AH | témoins | traitées par AH |
| M1/M2 | <10% | (42-100) | (0-56) | 22-100 |
| M3 | ― | ― | (69) 28-87 | 78 (42-90) |
| M5 | (<5) | (50-91) | ― | ― |

Le tableau III ci-dessus illustre les pourcentages de blastes CD15 positifs et CD14 positifs mesurés par cytométrie en flux, comme décrit en matériels et méthodes, parmi des blastes traités au AH ou des blastes témoins négatifs qui ont été colorés avec des anticorps conjugués à FITC dirigés contre CD15 (antigène spécifique de la voie granulocytaire) ou contre CD14 (antigène de la voie monocytaire). Ces pourcentages ont été déterminés par référence aux témoins isotypiques.
Des changements dans les MFI (valeur moyenne d'intensités de fluorescence, unités arbitraires) sont illustrés par la figure 2A, où est représenté le nombre de cellules CD14⁺ et CD15⁺ induites par utilisation d'AH sur des LAM1/2, 3, 4 et 5, en fonction de l'intensité de fluorescence (FITC), de gauche à droite, et de haut en bas : graphe CD14 LAM1/2, graphe CD15 LAM1/2, graphe CD15 LAM3, graphe CD14 LAM4, graphe CD15 LAM4, graphe CD14 LAMS (courbes noires : utilisation d'AH ; courbes grises, plus à gauche : témoins négatifs).
LAM3 : Dans les témoins négatifs, CD 15 était modérément exprimée (gamme de valeurs d'intensité moyenne de fluorescence (MFI) : 7 à 223) parmi 28 à 87% des blastes LAM3 (valeur moyenne 69%, exception faite de 2 échantillons qui se sont révélés CD15 négatifs). Après traitement au AH (activation de CD44), le % de cellules CD15⁺ a augmenté parmi 12 des 16 échantillons jusqu'à atteindre des valeurs de 42 à 90% (valeur médiane 78%), et de manière aussi élevée qu'avec l'acide tout-*trans*rétinoïque administré à 8 cas. De plus, les valeurs de MFI ont augmenté d'environ 3 fois *(cf.* Figure 2A), et sont aussi élevées, ou plus élevées, qu'avec l'acide tout- *trans*rétinoïque.
LAM5 : Pour six échantillons LAM5, CD14 n'a pas été détectable dans les témoins négatifs : dans cinq de ces échantillons, jusqu'à 50%-91% de cellules leucémiques se sont révélées CD14⁺ après traitement au AH (activation de CD44), *cf.* tableau III. De plus, deux autres échantillons LAM5 ont présenté des quantités significatives de CD14 dans les témoins négatifs : dans un de ces deux échantillons, le niveau de CD14 était augmenté de manière significative après traitement au AH (activation de CD44), avec une valeur de MFI atteignant 340 comparés à 102 dans les témoins négatifs *(cf.* figure 2A, graphe en bas à droite).
Ces résultats indiquent que les blastes LAM3 et LAM5 mûrissent vers les lignées granulocytaire et monocytaire respectivement. Pour 8 des 12 cas de LAM3 (66%), la maturation a été aussi marquée qu'après traitement avec l'acide tout-*trans*rétinoïque.
LAM1/2 : L'expression de CD14 tout comme celle de CD15 ont été mesurées sur les LAM1/2 puisque ces sous-types de cellules leucémiques myéloblastiques très immatures pourraient avoir conservé la capacité à se différencier vers les deux lignées granulocytaire et monocytaire, tout comme des cellules progénitrices myéloïdes immatures normales. L'expression de CD14 et CD15 (à la fois) a augmenté pour 6 des 8 cas de LAM1/2 après traitement au AH (activation de CD44). Pour CD14 tout comme pour CD15, la proportion de cellules exprimant ces antigènes de différenciation a augmenté : proportion de cellules positives CD14⁺ : moins de 10% dans les témoins, 42% à 100% dans les groupes traités ; proportion de cellules positives CD15⁺ : 0% à 56% dans les témoins, 22% à 100% dans les groupes traités *(cf.* tableau III)). L'intensité d'expression de ces antigènes (mean fluorescence intensity) apparaît également augmentée au regard des valeurs de MFI qui ont été multipliées d'un facteur de 2 environ *(cf.* figure 2A).

LAM4 : Finalement, les blastes LAM4 qui présentent spontanément des caractéristiques du phénotype granulo-monocytaire, se différencient également le long des lignées monocytaire et granulocytaire, comme le montre l'augmentation du % de cellules exprimant les antigènes de différenciateur CD14 et de CD15 (% de cellules positives multiplié d'un facteur de 2, *cf.* tableau III ) et l'augmentation des valeurs de MFI qui sont multipliées d'un facteur de 3 *(cf.* figure 2A).
Ainsi, tout comme le montre le test de réduction du NBT, la mesure de l'expression de CD14 et/ou CD15 indique que l'engagement de CD44 avec des molécules activatrices telles que l'acide hyaluronique induit la différenciation de tous les sous-types de blastes LAM.

Troisièmement, **l'induction de caractéristiques cytologiques spécifiques de cellules matures a été étudiée.** Ces résultats sont illustrés par la figure 2B qui présente six colorations de May-Grünwald-Giemsa, de gauche à droite photos du haut : témoins négatifs sur LAM3, blastes LAM3 traités 5 jours au AH, blastes LAM3 traités au AR (témoins positifs); photos du bas témoins négatifs sur LAM5, blastes LAM5 traités au AH, blastes LAM1 traités au AH (cellules NBT⁺).
Dans le cas de LAM3 (figure 2B, ligne du haut), les témoins négatifs (blastes non traités à l'extrémité gauche) présentent un phénotype promyélocytaire immature caractérisé par un rapport nucléo-cytoplasmique élevé, des nucléoles nombreuses et d'abondantes granulations cytoplasmiques azurophiles ; des corps d'Auer qui sont typiques des LAM M3 sont observés (flèche). Après traitement par AH (Figure 2B, photos centrale et de droite, ligne du haut), les LAM3 présentent un noyau segmenté, un faible rapport nucléo-cytoplasmique, de rares nucléoles, quelques granulations azurophiles, qui sont typiques des cellules granulocytaires différenciées (cellules de bandes et métamyélocytes). Ces caractéristiques sont semblables à celles des blastes traités à l'acide tout-transrétinoïque (photo de droite, ligne du haut), qui constituent le témoin positif de la différenciation LAM3. Des structures cytoplasmiques ressemblant à des structures endommagées de corps d'Auer peuvent être observées (flèche).
Dans le cas des blastes LAM5 (figure 2B, ligne du bas), les témoins négatifs (photo à gauche) présentent un rapport nucléo-cytoplasmique élevé, une chromatine finement réticulée avec de nombreuses nucléoles et une forme régulière, caractéristique des cellules monoblastiques immatures. Après traitement par AH (activation par CD44) (figure 2B, ligne du bas, photo centrale), les blastes LAM5 montrent une diminution du rapport noyau/cytoplasme, une diminution du nombre de nucléoles, une condensation de la chromatine, et des contours cytoplasmiques irréguliers, toutes ces caractéristiques étant typiques des monocytes matures.
Dans le cas des LAM1 (figure 2B, ligne du bas, photo à droite), les cellules NBT⁺ après traitement au AH, sont facilement reconnaissables par la coloration cytoplasmique sombre due à la réduction du NBT (x 100).
L'examen cytologique montre donc que les blastes LAM3 et LAM5 se différencient jusqu'aux stades terminaux de la granulopoïèse et de la monopoïèse, respectivement, après traitement au AH (activation de CD44). En effet, après traitement au AH, les blastes LAM3 présentent un noyau segmenté, quelques nucléoles et un nombre restreint de granulations azurophiles. Ces caractéristiques cytologiques, qui sont similaires à celles observées après traitement avec l'acide tout-*trans*rétinoïque sont caractéristiques de cellules granulocytaires différenciées (métamyélocytes et polymorphes segmentés). Par ailleurs, les LAM5 présentent, après traitement au AH, une diminution du rapport noyau/cytoplasme, une diminution du nombre de nucléoles, une condensation de la chromatine, et présentent des contours cytoplasmiques irréguliers, tous ces traits étant typiques de monocytes matures. Ces caractéristiques cytologiques corroborent les précédentes observations faites pour les LAM3 et les LAM5 selon les critères fonctionnels et antigéniques de différenciation. Aucun changement cytologique n'a été observé dans les LAM1/2 après traitement au AH, tel que réalisé, puisque les blastes de LAM1/2 sont très immatures : leurs différenciations terminales requièrent plus de 6 jours d'incubation et/ou l'action d'autres molécules différenciatrices telles que des cytokines pour achever leur différenciation jusqu'au stade terminal *in vitro*

### MODES D'ACTION D'AH

Nous montrons également que **l'intensité de la différenciation est directement liée à la dose de molécules activatrices utilisées, et au temps d'incubation mis en oeuvre.** La figure 3A illustre les résultats obtenus en incubant, comme décrit en méthodes, des blastes LAM5 en présence des concentrations indiquées de AH-12 (Figure 3A, graphe de gauche), ou en présence de 15 µg/ml de AH-12 pendant 3, 5 et 6 jours (Figure 3A, graphe de droite). Les données représentent la moyenne des intensités de fluorescence (MFI + écart-type) d'échantillons réalisés en triplicata et prélevés sur un élément représentatif de trois expériences.
L'intensité de différenciation induite par AH est donc liée à la dose de molécules activatrices utilisées : jusqu'à 15 µg/ml de AH, dans le cas LAM5, comme cela est montré en figuré 3A.

De plus, la capacité de ligands du CD44, et d'anticorps monoclonaux anti-CD44 en particulier, à inhiber fortement la liaison de AHh aux blastes LAM confirme que cette liaison de AHh est à tout le moins réalisée par l'intermédiaire de CD44. Ceci est illustré par la figure 3B où sont représentés, en fonction du log de l'intensité de fluorescence, et en identifiant pour chaque graphe les courbes de gauche à droite, le nombre de cellules témoins négatifs et de cellules traitées au AH-FITC seul (graphe de gauche), le nombre de cellules témoins négatifs, de cellules traitées au AH non marqué puis traité au AH-FITC et de cellules traitées an AH-FITC seul (graphe central), ainsi que le nombre de cellules témoins négatifs, de cellules traitées aux Acm anti-CD44 puis au AH-FITC, et de cellules traitées au AH-FITC seul (graphe de droite).
**La capacité différenciatrice de différents anticorps** monoclonaux (Acm) anti-CD44 a été testée *in vitro* sur des cellules prélevées sur des patients LAM comme il a été procédé pour AH. Tous les Acm anti-CD44 activateurs testés ne sont pas révélés capables d'induire une différenciation des blastes LAM dans ces conditions : parmi ceux-ci peut être cité l'Acm murin, Hermès 1, qui s'est révélé inefficace utilisé seul. D'antres Acm anti-CD44 activateurs se sont par contre révélés efficaces de manière équivalente à l'acide hyaluronique : l'Acm murin F10-44-2, par exemple. Il peut de plus être noté qu'à partir des Acm anti-CD44 à activité différenciatrice, il peut être réalisé, à l'aide des techniques classiques, des produits dont l'activité est comparable à celle de AH, ou de fragments de AH (AH6-AH20). Lorsque ces produits sont des Acm d'origine non humaine, il peut par exemple s'avérer avantageux de les humaniser (greffe de fragments CDR, Fab ou (Fab')₂ sur un anticorps matrice humain, par exemple) afin de prévenir des réactions antigéniques.

Concernant **les mécanismes** par lesquels AH exerce son action différenciatrice, il a été noté que, de manière remarquable, les Acm anti-CD44 à activité différenciatrice réagissent de manière croisée avec AH sur CD44, contrairement aux Acm anti-CD44 à activité non différenciatrice. Ces différents résultats mettent en évidence l'existence sur CD44 d'au moins un épitope spécifiquement impliqué dans la différenciation myéloïde. Cet épitope lié à la différenciation est localisé à l'intérieur du domaine de liaison de AH à CD44. Il peut être identifié par l'homme du métier à l'aide de tests ELISA, après digestion enzymatique de CD44 en fragments peptidiques. Nous présentons ci-dessous, délimitées par deux flèches, la séquence oligonucléotidique (SEQ ID N°7, séquence entre deux crochets, lignes du haut) dudit domaine de liaison de AH sur CD44, ainsi que sa séquence peptidique (SEQ ID N°8, séquence entre deux crochets, lignes du bas).

CAAGTTTTGGTGGCACGCAGCCTGGGG [ACTCTGCCTCGTGCCGCTGAGCCTGGCGCAGATCGATTTGAATATAAC

K F W W H A A W G [L C L V P L S L A Q I D L N I T

--CHO-

CTGCCGCTTTGCAGGTGTATTCCACGTGGAGAAAAATGGTCGCTACAGCATCTCTCGGACGGAGGCCGCTGACCTCTG

C R F A G V F H V E K N G R Y S I S R T E A A D L C

CAAGGCTTTCAATAGCACCTT GCCCACAATGGCCCAGATGGAGAAAGCTCTGAGCATCGGATTTGAGACCTGCAG

K A F N S T L P T M A Q M E K A L S I G F E T C R

--CHO--

GTATGGGTTCATAGAAGGGCATGTGGTGATTCCCCGGATCCACCCCAACTCCATCTGTGCAGCAAACAA] CAC

Y G F I E G H V V I P R I H P N S I C A A N N] T

--CHO--

AGGGGTGTACATCCTCACATACAACACCTCCCAGTATGACACATATTGCTTCAATGCTTC

G V Y I L T Y N T S Q Y D T Y C F N A S

--CHO-- --CHO-

Il a également pu être constaté que certains Acm anti-CD44 différenciateurs, lorsqu'ils sont utilisés en présence d'AHh ou de fragments d'AHh, sont capables de stimuler la liaison d'AHh (ou d'un fragment d'AHh) à sa cible, alors que d'autres au contraire s'y opposent et inhibent ainsi l'effet différenciateur (c'est le cas notamment de l'Acm J173).
Il a également pu être constaté que des Acm anti-CD44 non différenciateurs sont capables d'inhiber la liaison de AH à CD44. C'est le cas par exemple de Hermès 1. Ce résultat suggère que la reconnaissance par AH d'un épitope spécifique sur CD44 pourrait ne pas suffir à induire la différenciation observée.
Dans une minorité de cas de LAM (9/36), la différenciation n'a d'ailleurs pas été inductible par des ligands de CD44 tels que AH. Dans ces cas, l'analyse par cytométrie en flux a mis en évidence que les anticorps monoclonaux bloquant de manière croisée avec AH ne se lient pas à CD44. Cependant, CD44 était exprimé sur ces blastes puisque cette molécule a été marquée par des anticorps anti-CD44 conjugués à FITC. Ces résultats suggèrent que l'accessibilité de l'épitope(s) impliqué dans la différenciation pourrait être empêchée par une conformation particulière de la protéine CD44, ou par des motifs de glycosylation particuliers sur la molécule CD44, comme cela a pu être observé dans plusieurs cas de LAM.
Au-delà d'une ou plusieurs séquences de CD44 impliquées dans la différenciation, interviennent donc également, pour que la différenciation se déroule normalement, des contraintes de conformation de cette molécule.

### EVENEMENTS MOLECULAIRES

Pour aller plus avant dans les événements moléculaires liés à la différenciation induite par CD44, la dégradation de l'oncoprotéine PML-RARα chez les blastes LAM3 sous l'action de AH a été recherchée, comme cela a pu être reporté avec l'acide tout-*trans*rétinoïque. Dans ce but, des extraits protéiques de blastes de LAM3 (traités au AH, traités par AR ou blastes de témoins négatifs) ont été soumis à électrophorèse, transférés et confrontés avec un anticorps monoclonal spécifique anti-RARα comme décrit en matériels et méthodes. Les résultats sont illustrés par la figure 4A sur laquelle la bande à 110kD correspondant à PML-RARα (blastes témoins négatifs) apparaît fortement diminuée 24 heures après l'activation de CD44 par AH (piste 2), c'est-à-dire aussi efficacement qu'avec AR (acide tout-*trans*rétinoïque, piste 3), tandis que la protéine RARα de type sauvage (bande à environ 50kD) ne change pas.

Il a également été montré que la différenciation induite par CD44 implique
1) des phosphorylations de tyrosine, et
2) dans plusieurs cas, mais non dans tous, l'induction de l'expression de messagers de cytokine, c'est-à-dire des événements clés de la différenciation granulomonocytaire normale.
Premièrement, les inventeurs ont montré que la phosphorylation de protéines au niveau des tyrosines est cruciale dans la différenciation de LAM3 et LAM5 induite par CD44 (traitement au AH), puisque la génistéine, un inhibiteur spécifique des tyrosines kinases inhibe cette différenciation. Pour corroborer ce résultat, les inventeurs ont mis en évidence en utilisant un anticorps (6D12) conjugué à FITC, et la cytométrie en flux comme décrit en méthodes, que les phosphorylations de tyrosines intracellulaires sont déjà induites après une minute de traitement. Ceci est illustré par la figure 4C où est représentée l'intensité moyenne de fluorescence de tyrosines phosphorylées en fonction du temps, pour des blastes traités au AH (courbe du haut : 1 cas représentatif de LAM5), par comparaison à des blastes témoins négatifs (courbe du bas).
Deuxièmement, les cytokines suivantes sont connues pour être des acteurs spécifiques de l'induction d'une différenciation granulo-monocytaire normale GM-CSF (Facteur Stimulant la formation de Colonies Granulomonocytaire), G-CSF, et M-CSF. En utilisant la réaction par polymérase en chaîne semi-quantitative à l'aide de la transcriptase inverse (RT-PCR), 1 heure après l'activation de CD44 (traitement au AH), les transcrits M-CSF sont détectés chez les LAM M1/M2 (1 cas parmi 3), et un transcrit de M-CSF est détecté chez les LAM M5 (1 cas parmi 3). Ceci est illustré par la figure 4B qui représente des gels d'agarose obtenus après coloration au bromure d'éthidium des blastes LAM5 (photos de gauche : gel du haut M-CSF, gel du bas : marqueur GAPDH ; piste 1: témoins, piste 2 : traité au AH.
Ces inductions de différenciation ont donc impliqué des phosphorylations de tyrosines, puisqu'elles sont abrogées par le traitement avec la génistéine. Il est important de noter que dans de nombreux cas de LAM, les transcrits de GM-CSF, G-CSF et M-CSF ont été soit non détectés, soit exprimés de manière constitutive. Ceci suggère que ces cytokines ne sont pas impliquées de manière nécessaire dans la différenciation induite par CD44 (traitement au AH).

En conclusion, les inventeurs ont montré que la différenciation de blastes LAM peut être induite et/ou stimulée par AH ou ses fragments (à partir de AH-6), notamment via le CD44, pour tous les sous-types de LAM. Dans les LAM3, la différenciation induite par AH est comparable à celle obtenue avec l'acide rétinoïque. Les résultats présentés permettent notamment le développement de nouvelles thérapies pour la différenciation de LAM, notamment pour l'ensemble des sous-types M1 à M5, utilisant des molécules à structure d'acide hyaluronique, et/ou le ciblage de la molécule CD44 par d'autres molécules agonistes.

### EXEMPLE 2 : Réalisation d'un médicament destiné à stimuler ou induire la différenciation de cellules hématopoïétiques humaines.

L'acide hyaluronique (AH) utilisé dans l'exemple 1 ci-dessus a été purifié à partir de cordon ombilical humain (ICN Pharmaceuticals, Sigma). Pour la réalisation industrielle de médicaments, l'AH peut également être purifié à partir de tissus non humains : crête de coq (produit par la société Pharmacia sous la dénomination commerciale Healon) ou streptocoque, par exemple. Ces molécules d'AH présentent une masse moléculaire élevée. Or, ce sont les molécules AH de petite taille, qui peuvent être notamment obtenues par la digestion enzymatique de la forme de haut poids moléculaire, qui présentent les meilleures capacités différenciatrices selon l'invention. Une utilisation selon l'invention comprend avantageusement l'utilisation de molécules "fragments d'AH" composées de 3 à 10 disaccharidiques (AH-6 à AH-20, *cf.* Figure 1B). L'utilisation de ces petites molécules présente aussi un avantage pour la production pharmaceutique, car elles sont moins susceptibles d'être captées par le foie que les molécules AH de haut poids moléculaire.
Toute forme galénique peut être envisagée pour le médicament selon l'invention. Comme l'AH est très hydrosoluble, une préparation sous forme dissoute dans une solution saline équilibrée peut être aisément réalisée.
Comme les tissus hématopoïétiques (moelle osseuse, rate, ganglions) présentent une forte affinité pour AH, l'administration d'une telle solution peut être efficacement réalisée par injection par voie intraveineuse. Des doses d'administration de l'ordre de 1 à 10 mg d'AH/kg, avantageusement de l'ordre de 2 à 5 mg d'AH/kg, et notamment de l'ordre de 3mg d'AH/kg apparaissent avantageuses.
Si nécessaire, et notamment au vu des résultats du suivi de l'évolution de la maladie chez le patient, les doses peuvent être augmentées (en dose unitaire) et/ou répétées (dans le temps) : l'AH présente en effet l'avantage notable de ne pas être toxique, et de permettre ainsi une posologie d'administration parfaitement adaptée au patient considéré.
Enfin, il peut être bénéfique de diminuer la fixation importante de l'AH au niveau des sinus hépatiques. Dans cet organe, AH est fixé par la molécule de surface ICAM1, et non par le CD44. Le blocage préventif de cette fixation peut être éventuellement prévu en injectant de la chondroïtine sulfate qui saturerait les sites récepteurs ICAM-1.
La capacité du CD44 à fixer AH est variable, parfois faible à l'état constitutif, mais considérablement activable par certains anticorps monoclonaux (AcM) anti-CD44 de type activateur *(cf.* exemple 1). C'est pourquoi de tels AcMs peuvent, de manière particulièrement avantageuse, être incorporés au médicament selon l'invention à titre d'adjuvant(s) de la différenciation induite par AH. On peut donc prévoir de les injecter en même temps que AH. Sur la base des doses d'anticorps monoclonaux utilisées actuellement dans la thérapie cytotoxique des LAM, des doses de l'ordre de 5 à 10mg/m² d'AcM anti-CD44 activateurs apparaissent indiquées.

### EXEMPLE 3 : Action différenciatrice de l'acide l'hyaltironique sur les prolzénitetirs hématopoïétiques CD34⁺ de la moelle osseuse hmnaine normale.

### Matériel et Méthodes

Les cellules progénitrices hématopoïétiques CD34⁺ sont isolées de la moelle osseuse humaine normale par immunoadsorption sur billes magnétiques recouvertes d'anticorps anti-CD34. Ces cellules sont ensemencées à raison de 500 cellules/200µL dans du milieu de culture sans sérum (Stemcell medium) additionné des cytokines IL-1 (100U/mL), IL-3 (2ng/mL) et SCF (10ng/mL) et de 50µg/mL d'AH (molécules composées de 10 à 50 saccharides). L'AH n'est pas ajouté aux groupes contrôlés. Après 7 jours d'incubation à 37°C l'expression des antigènes de différenciation CD15 (granulocytaire) et CD14 (monocytaire) est analysée par cytométrie en flux.

La même expérience est réalisée avec des cellules hématopoïétiques CD34⁺ isolées du sang du cordon ombilical.

### Résultats

On mesure par intensité de fluorescence le nombre de cellules CD15⁺ et CD14⁺ dans les différents traitements. Les résultats obtenus pour les cellules hématopoïétiques CD34⁺ isolées de moelle oseuse sont illustrés sur la figure 5. Les résultats obtenus pour les cellules hématopoïétiques isolées du sang du cordon ombilical sont comparables. Dans les deux cas, on observe une proportion significativement supérieure de cellules CD 15⁺ et CD 14⁺ dans les lots traités par les fragments d'AH.

### Conclusion

Les fragments d'AH stimulent la différenciation des cellules progénitrices hématopoïétiques CD34⁺ isolées de la moelle osseuse humaine, c'est-à-dire des cellules souches très immatures (CD15⁻, CD14⁻), et cela non seulement selon la voie monocytaire, mais également selon la voie granulocytaire.

### EXEMPLE 4 : Fragments d'AH présentant plus de 10 unités disaccharidiques

En procédant de manière comparable à l'exemple 1, il peut être observé que nous avons montré que des fragments d'AH composés de 20 à 100 unités saccharides et utilisés à raison de 50µg/mL induisent la différenciation terminale des blastes LAM1 et LAM2. Cette différenciation est démontrée :
- par l'augmentation de l'expression des antigènes de différenciation CD14 et CD15 :
   LAM1: 13% de cellules CD14⁺ dans le groupe traité comparé à moins de 5% dans le groupe contrôle; 72% de cellules CD15⁺ (intensité de fluorescence relative de 56) dans le groupe traité comparé à 55% de cellules CD15⁺ (intensité de fluorescence relative de 21 ) dans le groupe contrôle.
   LAM2 : 35% de cellules CD14⁺ dans le groupe traité comparé à moins de 5% dans le groupe contrôle.
- par l'induction de cellules NBT⁺ : 50% dans le groupe traité (LAM1) comparé à moins de 5% dans le groupe contrôle.
- par l'induction de caractéristiques cytologiques spécifiques de monocytes matures.

## Revendications

1. Utilisation d'un polymère comprenant une quantité efficace d'unités disaccharidiques composées chacune d'une molécule à structure de N-acétyl-D-glucosamine liée par liaison O-glucosidique β1,4 à une molécule à structure d'acide glucuronique pour la fabrication d'un médicament destiné à induire ou stimuler la différenciation de cellules choisies parmi des cellules leucémiques et des cellules souches CD14⁻CD15⁻.

2. Utilisation selon la revendication 1, **caractérisée en ce que** ladite quantité efficace correspond à un nombre d'unités disaccharidiques supérieur ou égal à 3.

3. Utilisation selon l'une quelconque des revendications précédentes, **caractérisée en ce que** ladite quantité efficace correspond à un nombre compris entre 3 et 10.

4. Utilisation selon l'une quelconque des revendications précédentes, **caractérisée en ce que** ladite quantité efficace correspond à un nombre compris entre 10 et 100.

5. Utilisation selon l'une quelconque des revendications précédentes, **caractérisée en ce que** ledit polymère est choisi parmi le groupe constitué par l'acide hyaluronique et les fragments de cet acide.

6. Utilisation selon l'une quelconque des revendications précédentes, **caractérisée en ce que** ledit polymère est utilisé pour le fabrication dudit médicament à une dose unitaire comprise entre 1 et 10 mg/kg, avantageusement entre 2 et 5 mg/kg, notamment de l'ordre de 3 mg/kg.

7. Utilisation selon l'une quelconque des revendications précédentes, **caractérisée en ce que** ledit polymère est utilisé sous forme de solution, préférablement de solution injectable par voie intraveineuse.

8. Utilisation selon l'une quelconque des revendications précédentes, **caractérisée en ce qu'**elle comprend, en outre, l'utilisation d'un composé adjuvant capable de stimuler la liaison dudit polymère à une cible cellulaire, tel qu'un anticorps monoclonal anti-CD44, ou un fragment d'un tel anticorps.

9. Utilisation selon l'une quelconque des revendications précédentes, **caractérisée en ce qu'**elle comprend, en outre, l'utilisation d'un composé capable de prévenir la liaison dudit polymère à une cible cellulaire inappropriée, et en particulier, un anticorps monoclonal anti-ICAM1 ou un fragment d'un tel anticorps.

10. Utilisation selon l'une quelconque des revendications précédentes, **caractérisée en ce que** lesdites cellules leucémiques sont des cellules de leucémie aiguë myéloblastique LAM1/2 et/ou LAM3 et/ou LAM4 et/ou LAM5 et/ou LAM6 et/ou LAM7.

11. Utilisation selon la revendication 1, **caractérisée en ce que** ledit polymère est sous la forme d'un agent mimétique ou agoniste.

12. Utilisation selon la revendication 11, **caractérisée en ce que** l'agent mimétique ou agoniste est un anticorps humain ou humanisé.

## Claims

1. Use of a polymer comprising an effective quantity of disaccharide units each composed of a molecule with an N-acetyl-D-glucosamine structure bound by an O-glucoside β1,4 bond to a molecule with a glucuronic acid structure for the manufacture of a medicament intended to induce or stimulate the differentiation of cells chosen from leukemia cells and cells of strains CD14⁻CD15⁻.

2. Use according to claim 1, **characterized in that** said effective quantity corresponds to a number of disaccharide units greater than or equal to 3.

3. Use according to any one of the previous claims, **characterized in that** said effective quantity corresponds to a number comprised between 3 and 10.

4. Use according to any one of the previous claims, **characterized in that** said effective. quantity corresponds to a number comprised between 10 and 100.

5. Use according to any one of the previous claims, **characterized in that** said polymer is chosen from the group constituted by hyaluronic acid and the fragments of this acid.

6. Use according to any one of the previous claims, **characterized in that** said polymer is used for the manufacture of said medicament at a single dose comprised between 1 and 10 mg/kg, advantageously between 2 and 5 mg/kg, in particular of the order of 3 mg/kg.

7. Use according to any one of the previous claims, **characterized in that** said polymer is used in the form of a solution, preferably a solution which can be injected by intravenous route.

8. Use according to any one of the previous claims, **characterized in that** it comprises, in addition, the use of an adjuvant compound capable of stimulating the bond of said polymer to a target cell, such as an anti-CD44 monoclonal antibody or a fragment of such an antibody.

9. Use according to any one of the previous claims, **characterized in that** it comprises, in addition, the use of a compound capable of preventing the binding of said polymer to an inappropriate target cell, and in particular, an anti-ICAM1 monoclonal antibody or a fragment of such an antibody.

10. Use according to any one of the previous claims, **characterized in that** said leukemia cells are acute myeloblastic leukemia cells LAM1/2, and/or LAM3 and/or LAM4 and/or LAM5 and/or LAM6 and/or LAM7.

11. Use according to claim 1, **characterized in that** said polymer is in the form of a mimetic agent or agonist.

12. Use according to claim 11, **characterized in that** the mimetic agent or agonist is a human or humanized antibody.

## Patentansprüche

1. Verwendung eines Polymers mit einer wirksamen Quantität von Disaccharideinheiten, die jede aus einem Molekül mit N-Acetyl-D-Glucosaminstruktur bestehen, das durch β-1,4-O-Glykosid-Bindung mit einem Molekül mit Glucuronsäurestruktur verbunden ist, zur Herstellung eines Medikaments, das bestimmt ist zur Induktion oder Stimulation der Differenzierung von Zellen, die ausgewählt sind aus leukämischen Zellen und Stammzellen CD14⁻CD15⁻.

2. Verwendung nach Anspruch 1, **dadurch gekennzeichnet, daß** die wirksame Quantität einer Anzahl von Disaccharideinheiten von mehr als oder gleich 3 entspricht.

3. Verwendung nach einem der vorangehenden Ansprüche, **dadurch gekennzeichnet, daß** die wirksame Quantität einer Zahl zwischen 3 und 10 entspricht.

4. Verwendung nach einem der vorangehenden Ansprüche, **dadurch gekennzeichnet, daß** die wirksame Quantität einer Zahl zwischen 10 und 100 entspricht.

5. Verwendung nach einem der vorangehenden Ansprüche, **dadurch gekennzeichnet, daß** das Polymer ausgewählt ist aus der Gruppe bestehend aus Hyaluronsäure und Fragmenten dieser Säure.

6. Verwendung nach einem der vorangehenden Ansprüche, **dadurch gekennzeichnet, daß** das Polymer verwendet wird zur Herstellung des Medikaments mit einer Dosiseinheit zwischen 1 und 10 mg/kg, vorteilhafterweise zwischen 2 und 5 mg/kg besonders in der Größenordnung von 3 mg/kg.

7. Verwendung nach einem der vorangehenden Ansprüche, **dadurch gekennzeichnet, daß** das Polymer in Form einer Lösung, vorzugsweise einer intravenös injizierbaren Lösung verwendet wird.

8. Verwendung nach einem der vorangehenden Ansprüche, **dadurch gekennzeichnet, daß** sie außerdem die Verwendung einer verstärkenden Verbindung umfaßt, weiche die Bindung des Polymers an sein zelluläres Ziel verstärken kann, wie ein Anti-CD44-monoklonaler Antikörper oder ein Fragment eines solchen Antikörpers.

9. Verwendung nach einem der vorangehenden Ansprüche, **dadurch gekennzeichnet**, t, daß sie außerdem die Verwendung einer Verbindung umfaßt, welche die Bindung des Polymers an ein ungeeignetes zelluläres Ziel verhindern kann, und besonders einen Anti-ICAM1-monoklonalen Antikörper oder ein Fragment eines solchen Antikörpers.

10. Verwendung nach einem der vorangehenden Ansprüche, **dadurch gekennzeichnet, daß** die leukämischen Zellen Zellen der akuten myeloblastischen Leukämie LAM1/2 und/oder LAM3 und/oder LAM4 und/oder LAM5 und/oder LAM6 und/oder LAM7 sind.

11. Verwendung nach Anspruch 1, **dadurch gekennzeichnet, daß** das Polymer in Form eines mimetischen Agens oder Agonisten vorliegt.

12. Verwendung nach Anspruch 11, **dadurch gekennzeichnet, daß** das mimetische Agens oder Agonist ein humaner oder humanisierter Antikörper ist.
